# EUROPÄISCHE PATENTSCHRIFT

(11) **EP 1 148 824 B1**
(45) Veröffentlichungstag und Bekanntmachung des Hinweises auf die Patenterteilung: **08.08.2007**
(21) Anmeldenummer: 00901020.8
(22) Anmeldetag: 31.01.2000
(51) Int. Cl.: A61B 17/16

(54) **VORRICHTUNG MIT FLEXIBLER WELLE ZUR GEWINNUNG VON KNOCHENSPÄNEN**
DEVICE WITH A FLEXIBLE SHAFT FOR REMOVING BONE GRAFTS
DISPOSITIF A ARBRE FLEXIBLE POUR PRELEVER DES COPEAUX D'OS

(30) Priorität: 02.02.1999 DE 29901724 U
(43) Veröffentlichungstag der Anmeldung: 31.10.2001
(73) Patentinhaber: Synthes GmbH, 4436 Oberdorf (CH)
(72) Erfinder: STEINER, Béatrice, CH-6330 Cham (CH); HEHLI, Markus, CH-7276 Frauenkirch (CH); AEBI, Max, McGill University, Montreal, Quebec H3A 1A1 (CA); STEFFEN, Thomas, Montreal, DC H2Y 2B7 (CA)
(74) Vertreter: Lusuardi, Werther
(86) Internationale Anmeldenummer: PCT/CH2000/000047
(87) Internationale Veröffentlichungsnummer: WO 2000/045713

(56) Entgegenhaltungen:
- WO-A-96/39956
- WO-A-97/38635
- WO-A-97/39685
- US-A- 4 646 738
- US-A- 5 322 505
- US-A- 5 403 317
- US-A- 5 556 399
- US-A- 5 569 284

## Beschreibung

Die Erfindung bezieht sich auf eine Vorrichtung zur Gewinnung von Knochenspänen gemäss dem Oberbegriff des Patentanspruchs 1.

Das Implantieren von körpereigenem Knochenmaterial bleibt die effizienteste Behandlungsmethode bei Nichtheilen eines gebrochenen Knochens, Pseudoarthrosis und zur Optimierung der Erfolgsrate bei Arthrodesis. Die Verwendung von körpereigenem Knochenmaterial ist sicherer und wirksamer als die Verwendung künstlich hergestellter Hydroxyapatit-Materialien oder körperfremder Knochenspäne, bedingt jedoch einen zusätzlichen Eingriff am Körper des Patienten. Dies kann durch ein begrenztes Eindringen und durch den Gebrauch einer zylindrischen Nadel, wie sie zur Entfernung von Knochenmaterial für Diagnosezwecke verwendet wird, minimiert werden. Diese Technik ist jedoch kompliziert und gefährlich, da keine genaue Kontrolle gewährleistet ist. Meist wird deshalb die Spongiosa durch einen grösseren Hautschnitt und aus einer grossen Öffnung am Beckenkamm herausgemeisselt. Spezielle Knochenspan-Sammelinstrumente gestatten eine sichere und rasche Gewinnung von körpereigenen Knochenspänen durch einen kleinen Hauteinschnitt, was die Unannehmlichkeiten und Verletzungen des Patienten minimiert. Diese Vorrichtungen entfernen zuverlässig das Knochenmaterial und können mit einer Bohrmaschine angewendet werden, wodurch eine grössere Menge und eine bessere Kontrollmöglichkeit gewonnen werden sowie ein versehentliches Durchstossen durch die Kortikalis minimiert wird. Diese sichere und wirksame Technik ermöglicht, körpereigene Knochenspäne für Fusionen, Pseudoarthrosis und Knochenbrüche mit einer minimalen Verletzung des Spenders zu gewinnen. Die Entfernung der Knochenspäne am Körper des Patienten wird üblicherweise am Beckenknochen vorgenommen. Ebenfalls brauchbares Knochenmaterial lässt sich proximal an der Ulna oder distal am Radius gewinnen.

Eine solche Vorrichtung zur Gewinnung von Knochenmaterial ist beispielsweise aus der WO 97/39685 YUAN bekannt. Diese bekannte Vorrichtung umfasst einen starren hohlzylindrischen und durchsichtigen Schaft, worin die Knochenspäne gesammelt werden und wegen der Durchsichtigkeit durch einen einzigen Blick die Menge der gesammelten Knochenspäne erkennbar ist, einen Schneidkopf am eine Ende und Mittel zur Aufnahme eines Drehmomentes am anderen Ende des Schaftes. Die Vorrichtung wird einfach in den Knochen eingedreht, wodurch mittels des Schneidkopfes der Knochen zerspant und abgetragen wird. Die Knochenspäne werden im Hohlraum im Schaft aufgenommen und gesammelt. Bei Gebrauch werden die gesammelten Knochenspäne mittels eines Kolbens, welcher vom dem Schneidkopf entgegengesetzten Ende des Schaftes in dessen Hohlraum eingeschoben wird, aus dem Schaft entnommen. Rotativ angetrieben werden kann die Vorrichtung von Hand oder maschinell.

Eine weitere solche Vorrichtung zur Gewinnung von Knochenmaterial ist aus der US 5,556,399 HUEBNER bekannt. Auch diese bekannte Vorrichtung umfasst einen Bohrkopf mit einem daran anschliessenden starren hohlzylindrischen Schaft, worin die Kochenspäne gesammelt werden und anschliessend durch einen vom Bohrkopf her einzuführenden handbedienten Kolben wieder aus dem Hohlraum entnommen werden.

Eine Methode und eine Vorrichtung zur Gewinnung von Gewebe ist aus der US 5,403,317 BONUTTI bekannt. Diese bekannte Erfindung umfasst eine Vorrichtung zur perkutanen Gewebegewinnung und besteht aus einem bezüglich Biegung flexiblen Bohrerschaft und Mitteln zum Antrieb des Schaftes. Zum Herausschneiden von Gewebefragmenten aus dem Gewebe ist vorne am Schaft eine Schneidspitze angebracht. Die Gewebefragmente werden während des Schneidvorganges mittels eines Unterdruckes durch den Schaft zu einem Ort ausserhalb des Körpers gesaugt.

Eine weitere Vorrichtung zur Gewinnung von Knochenspänen ist aus der US 4,646,738 TROTT bekannt. Diese bekannte Vorrichtung umfasst ein äusseres rohrförmiges Teil und ein inneres rohrförmiges Teil, wobei am einen Ende des inneren rohrförmigen Teiles ein Schneidwerkzeug angebracht ist. Das innere rohrförmige Teil ist innerhalb des äusseren rohrförmigen Teiles rotierbar gelagert, während das äussere rohrförmige Teil aus einem deformierbaren Material hergestellt und selektiv biegbar ist.

Wiederum eine andere Vorrichtung zur Gewinnung von Knochenspänen ist aus der WO 96/39956 AUST bekannt. Diese bekannte Vorrichtung umfasst einen dünnwandigen, hohlzylindrischen Schaft, welcher aussen mit einer Spiralfeder umgeben ist

Nachteilig bei allen diesen bekannten Vorrichtungen ist die Gefahr, dass mit den bekannten Vorrichtungen wegen der Starrheit des Schaftes bezüglich Torsion beim Ausräumen der Spongiosa zwischen der Kortikalis die härtere Kortikalis geschnitten oder durchbrochen wird.

Hier will die Erfindung Abhilfe schaffen. Der Erfindung liegt die Aufgabe zugrunde, eine Vorrichtung zu entwickeln, welche es in einfacher Weise gestattet, die Spongiosa zwischen der Kortikalis auszuräumen, ohne dadurch die härtere Kortikalis zu schneiden oder durch diese hindurchzubrechen.

Spongiosa zwischen der Kortikalis auszuräumen, ohne dadurch die härtere Kortikalis zu schneiden oder durch diese hindurchzubrechen.

Die Erfindung löst die gestellte Aufgabe mit einer Vorrichtung zur Gewinnung von Knochenspänen, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass die elastische Verformbarkeit des Schaftes und ein nicht zu scharfkantig ausgebildeter Schneidkopf gestatten, die Spongiosa zwischen der Kortikalis auszuräumen, ohne dadurch die härtere Kortikalis zu schneiden oder durch diese hindurchzubrechen.

Die erfindungsgemässe Vorrichtung umfasst ein hohlzylindrisches Schneidwerkzeug mit einem hohlzylindrischen Schneidkopf, welcher verschieden gestaltete Bohrspitzen und Schneidkanten aufweisen kann, einem hohlzylindrischen Schaft mit Mitteln zum Einspannen des Schaftes in eine Antriebsvorrichtung, und eine Antriebsvorrichtung, welche beispielsweise aus einer Universalbohrmaschine bestehen kann. Die hohlzylindrische Ausgestaltung des Schneidkopfes und des Schaftes ermöglicht eine Aufnahme der vom Schneidkopf abgetragenen Knochenspäne aus der Spongiosa in der Bohrung im Innern der Hohlzylinder. Der an den Schneidkopf anschliessende Schaft ist bezüglich Torsion und Biegung elastisch verformbar. In der Bohrung ist ein Kunststoff -oder Grummischlauch eingeführt.

Diese Verformbarkeit lässt sich durch eine Ausführung des Schaftes als spiralförmig gewickeltes Metallblechband, als mit einer Drahtarmierung verstärkter Kunststoff- oder Gummischlauch oder auch als Metallrohr mit balgartiger Seitenwand herstellen.

Die Bohrspitze des Schneidkopfes ist vorzugsweise als Sektor einer Kugelkalotte mit einer Schneidkante ausgeführt. Die durch diese Ausgestaltung des Schneidkopfes erreichten Vorteile sind darin zu sehen, dass ein nicht zu scharfkantig oder mit scharfen Ecken ausgebildeter Schneidkopf gestattet, die Spongiosa zwischen der Kortikalis auszuräumen, ohne dadurch die härtere Kortikalis zu schneiden oder durch diese hindurchzubrechen.

Eine spezielle Ausführungsform des Schneidkopfes besteht darin, dass die Bohrspitze des Schneidkopfes kugelkalottenförmig mit mindestens zwei koaxial und radial zur Längsachse sich in den Hohlraum erstreckenden Durchgangsöffnungen ausgebildet ist, wobei an den Kanten der Durchgangsöffnungen Schneidkanten zum Abtragen von Knochenspänen angebracht sind und die abgetragenen Knochenspäne durch die Durchgangsöffnungen in den Hohlraum des Schneidkopfes förderbar sind.

Andere Ausführungsformen der Bohrspitze sind als Kegelsektoren mit Schneidkanten oder als hohlzylindrische Fräser mit stirnseitigen Schneidezähnen denkbar.

Die Verbindung zwischen Schneidkopf und Schaft ist als lösbare oder feste Verbindung denkbar, wobei eine lösbare Verbindung einen kleineren Werkzeugsatz ermöglicht. Als lösbare Verbindung sind Schraubverbindungen, radiale Stiftschrauben oder radiale Stiftverbindungen möglich.

In einer bevorzugten Ausführungsform der erfindungsgemässen Vorrichtung ist an der Antriebsvorrichtung ein mit einem Vakuum beaufschlagter Behälter so angebracht, dass das hohlzylindrische Schneidwerkzeug mit seinem vom Schneidkopf entfernten Ende in den Behälter mündet, wodurch die Knochenspäne mittels des Vakuums auf einfache Weise durch das hohlzylindrische Schneidwerkzeug in den Behälter förderbar sind und im Behälter gesammelt werden können. Zum Absaugen der Knochenspäne ist am Behälter ein Stutzen für den Anschluss eines Vakuumschlauches angebracht. Durch das so angelegte Vakuum werden die Knochenspäne durch eine oder mehrere Durchgangsöffnungen im Schneidkopf in die Bohrung im Schaft gesaugt und von dort durch das gesamte Schneidwerkzeug hindurch in den Behälter gefördert. Damit die Knochenspäne nicht in den Vakuumschlauch geraten, ist im Behälter eine Abscheidevorrichtung zum Abscheiden der Knochenspäne aus dem Luftstrom angebracht. Dieser Abscheider kann als Filter, Sieb, Prallplatte oder Zyklon ausgeführt sein.

Diese Ausgestaltung der Vorrichtung gestattet, dass die Knochenspäne ohne Entfernung des Bohrwerkzeuges aus dem Bohrloch von der Bohrspitze durch den Schaft hindurch wegförderbar sind und in einem direkt an das Bohrwerkzeug anschliessenden Behälter gesammelt werden können. Der Behälter kann von der Vorrichtung demontiert werden und bei Bedarf sind die Knochenspäne einfach aus dem Behälter entnehmbar.

Das am Behälter angelegte Vakuum umfasst einen Druckbereich von 0 bar bis 1 bar, vorzugsweise jedoch einen Druckbereich von 0,2 bar bis 0,8 bar.

Zur Abdichtung der unter Vakuum stehenden Bohrung im Schaft des Schneidwerkzeuges wird vorzugsweise in diese Bohrung ein Gummi- oder Kunststoffschlauch eingezogen.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Ansicht des Schneidwerkzeuges mit dem flexiblen Schaft gemäss einer Ausführungsform der erfindungsgemässen Vorrichtung;
Fig. 2 eine perspektivische Darstellung des Schneidkopfes mit dem flexiblen Schaft einer Ausführungsform der erfindungsgemässen Vorrichtung;
Fig. 3 eine schematische Darstellung einer bevorzugten Ausführungsform der erfindungsgemässen Vorrichtung; und
Fig. 4 eine schematische Darstellung einer weiteren bevorzugten Ausführungsform der erfindungsgemässen Vorrichtung.

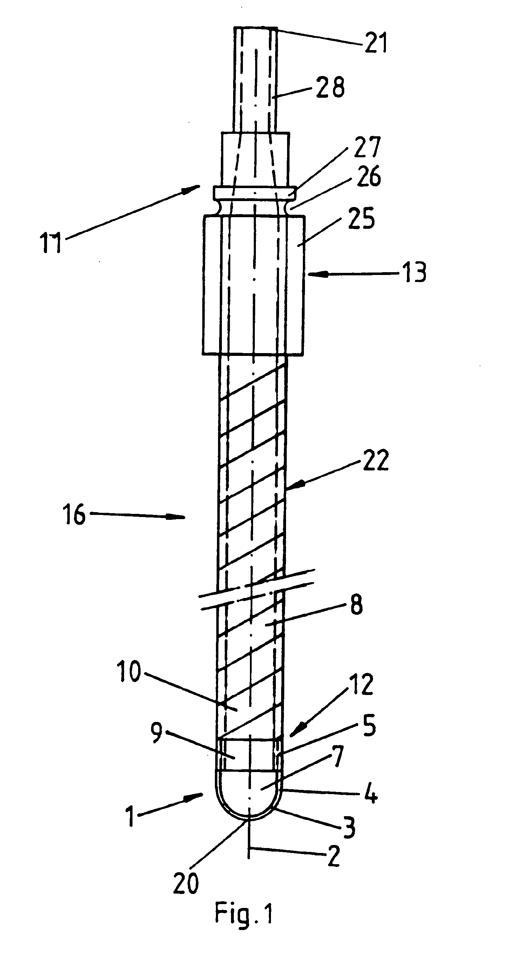

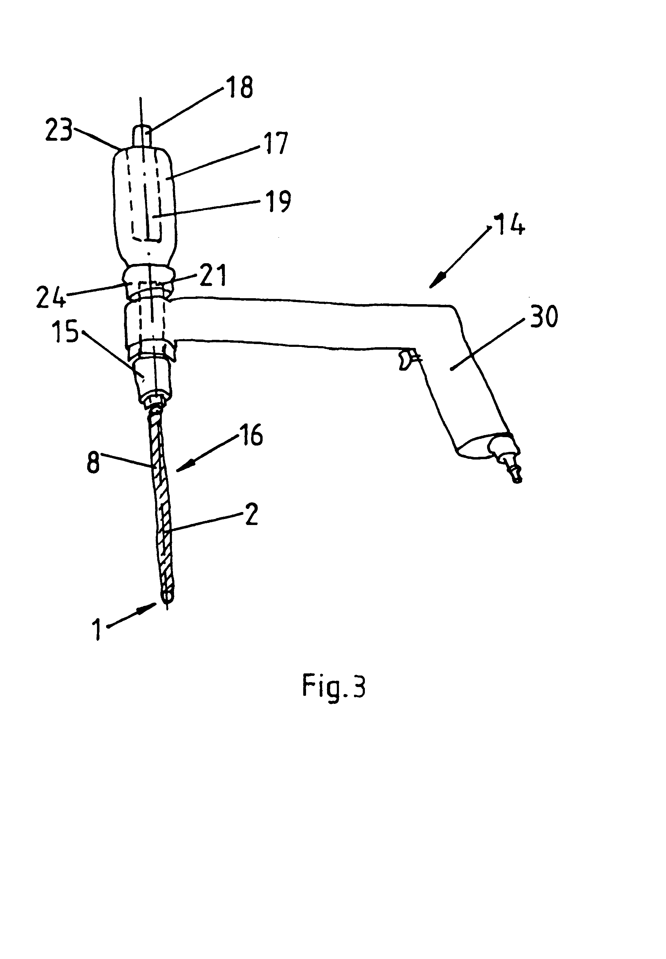

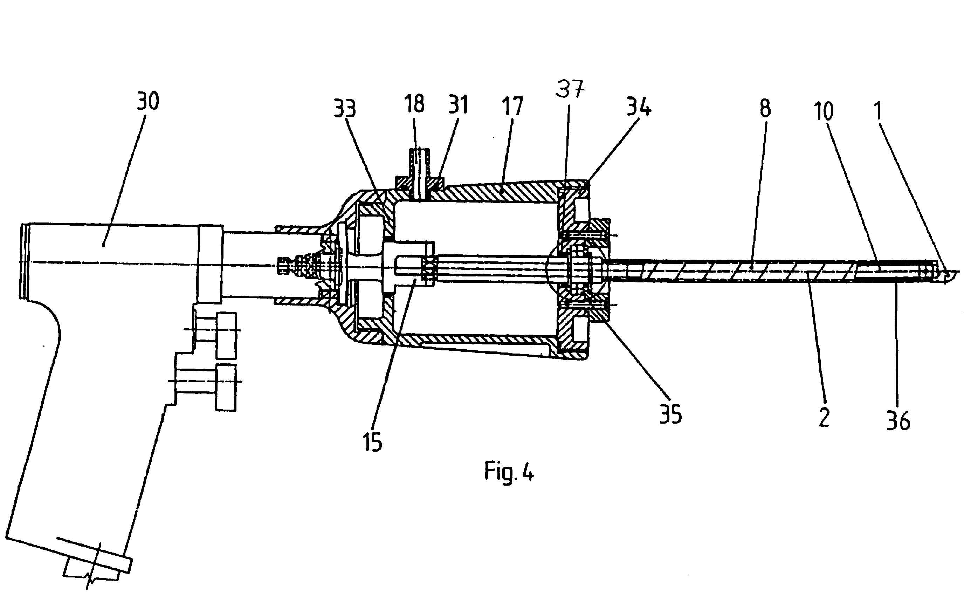

In Fig. 1 ist das Schneidwerkzeug 16 mit Schneidkopf 1 und Schaft 8 dargestellt. Der Schaft 8 umfasst einen bezüglich Torsion und Biegung elastisch verformbaren Teil 22 und einen mit Mitteln 13 zur Aufnahme eines Drehmomentes versehenen, vom Schneidkopf 1 entfernten Teil 11. Die Mittel 13 bestehen aus einem Abschnitt 25 mit Aussensechskant und einem daran anschliessenden zylindrischen Abschnitt 27 mit einer Nute 26. Die beiden Abschnitte 25 und 27 lassen sich in entsprechende Einspannmittel 15 (Fig. 3) an einem Antriebsmittel 14 (Fig. 3) einspannen, wobei der Schaft 8 mittels der Nute 26 axial und durch den Aussensechskant rotativ im Einspannmittel 15 (Fig. 3) lösbar fixierbar ist. Die Bohrung 10 im hohlzylindrischen Schaft 8 durchdringt den Schaft 8 in Richtung der Längsachse 2 vom Schneidkopf 1 bis zu dem vom Schneidkopf 1 entfernten Ende 21 des Schaftes 8, so dass die vom Schneidkopf 1 abgetragenen Knochenspäne entlang der Längsachse 2 durch das ganze Schneidwerkzeug 16 förderbar sind. Zur Fixation des Schneidkopfes 1 am Schaft 8 sind Feststellschrauben oder beispielsweise auch Federstifte zwischen Schaft 8 und Schneidkopf 1 denkbar. Der elastisch verformbare Teil 22 des Schaftes 8 ist aus einem spiralförmig gewickelten Metallstreifen gefertigt, wobei in der Bohrung 10 ein Gummi- oder Kunststoffschlauch 36 (Fig. 4) eingelegt ist, welcher gegenüber der Umgebung einen luftdichten Abschluss in der Bohrung im Schlauch 36 gewährleistet.

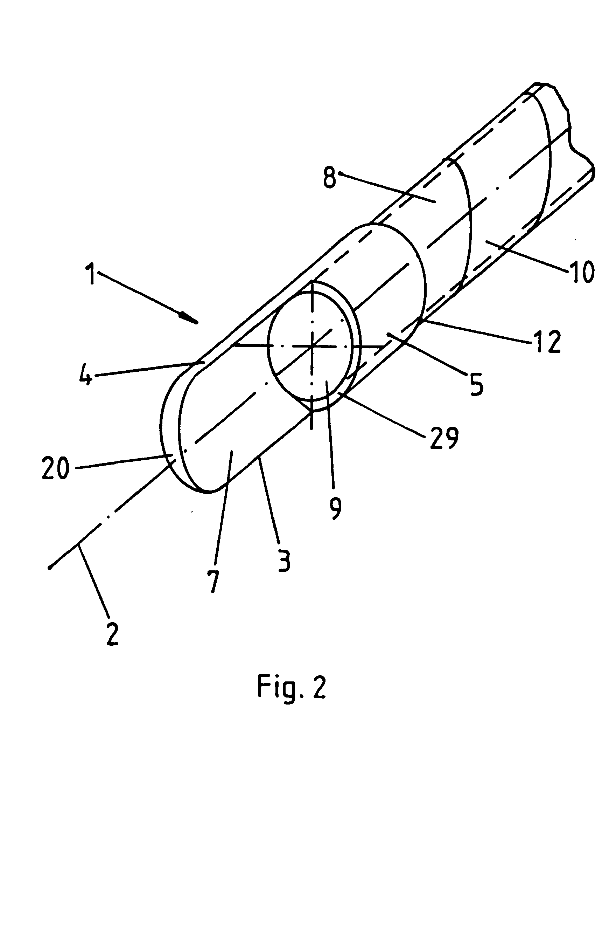

Fig. 2 zeigt eine Ausführungsform des Schneidkopfes 1. Der Schneidkopf 1 ist als Hohlzylinder mit einer Längsachse 2 und einer Bohrspitze 20 ausgeführt und umfasst einen vorderen an die Bohrspitze 20 anschliessenden Abschnitt 4 und einen hinteren von der Bohrspitze 20 entfernten Abschnitt 5. Der vordere Abschnitt 4 besteht aus einem Hohlzylinder mit einer als Kugelkalottensektor ausgebildeten Bohrspitze 20, wobei die im Querschnitt rechtwinklig zur Längsachse 2 betrachtete Seitenwand des vorderen Abschnitts 4 nur einen Kreisringsektor einschliesst, so dass eine radial zum hohlzylindrischen Teil und axial zur Bohrspitze 20 verlaufende Durchgangsöffnung 7 entsteht. Die Seitenwand des vorderen Abschnittes 4 ist von der Bohrspitze 20 bis zum hinteren Abschnitt 5 gegen die Durchgangsöffnung 7 hin als Schneidkante 3 ausgebildet. Wird der rotierende Schneidkopf 1 in den Knochen gebohrt, so werden durch die Schneidkanten 3 Knochenspäne abgetragen und gelangen durch die Durchgangsöffnung 7 in den Hohlraum 9 des Schneidkopfes 1 und werden von dort durch die Bohrung 10 im Schaft 8 gefördert.

In Fig. 3 ist eine bevorzugte Ausführungsform der erfindungsgemässen Vorrichtung dargestellt. Das Schneidwerkzeug 16, welches zur Gewinnung der Knochenspäne dient, besteht aus einem Schneidkopf 1 mit einem sich entlang einer Längsachse 2 erstreckenden hohlzylindrischen Schaft 8. Dieser Schaft 8 ist in Einspannmitteln 15 einer als Antriebsmittel 14 dienenden Universalbohrmaschine 30 axial und rotativ fixiert. Durch die Antriebsmittel 14 wird dem Schaft 8 mit dem Schneidkopf 1 eine Rotationsbewegung um die Längsachse 2 aufgeprägt, wodurch sich der Schneidkopf 1 in den Knochen bohrt und die zu sammelnden Knochenspäne abträgt. Der Schaft 8 ist vom Schneidkopf 1 bis zu seinem vom Schneidkopf 1 entfernten Ende 21 hohlzylindrisch ausgeführt, so dass die Knochenspäne entlang der gesamten Länge des Schneidwerkzeuges 16 förderbar sind. Ebenfalls an den Antriebsmitteln 14 angebracht ist ein als Behälter 17 für die Knochenspäne dienendes Gefäss. Der Behälter 17 ist koaxial zur Längsachse 2 mit seinem vorderen Ende 24 so an den Antriebsmitteln 14 lösbar befestigt, dass das vom Schneidkopf 1 entfernte Ende 21 des Schneidwerkzeuges 16 gegenüber der Umgebung luftdicht in den Behälter 17 mündet. An seinem vom Schaft 8 entfernten Ende 23 ist der Behälter 17 mit einem Stutzen 18 versehen, woran sich ein Vakuumschlauch (nicht gezeichnet) anschliessen lässt. Durch das Vakuum im Schlauch wird der Behälter 17 ebenfalls evakuiert, wodurch im Innern des hohlzylindrischen Schneidwerkzeuges 16 ein Unterdruck entsteht und somit die vom Schneidkopf 1 abgetragenen Knochenspäne durch das Innere des Schaftes 8 gesaugt werden und in den Behälter 17 gelangen, wo sie dann in der Folge gesammelt werden können. Damit die Knochenspäne nicht durch das Vakuum mit in den Vakuumschlauch gerissen werden, ist im Behälter 17 eine Abscheidevorrichtung 19, welche in der bevorzugten Ausführungsform als Filter ausgestaltet ist, so angebracht, dass die Knochenspäne nicht durch den Stutzen 18 austreten können.

In Fig. 4 ist eine weitere bevorzugte Ausführungsform der erfindungsgemässen Vorrichtung dargestellt. Diese hier dargestellte Ausführungsform der Vorrichtung unterscheidet sich von der in Fig. 3 dargestellten Ausführungsform nur darin, dass das Schneidwerkzeug 16 durch den koaxial zur Längsachse 2 angeordneten Behälter 17 durchgeht und die Mittel 13 zur Aufnahme eines durch die Universalbohrmaschine 30 abgegebenen Drehmomentes im Bereich des vom Schneidkopf 1 entfernten Behälterbodens 33 mit der Universalbohrmaschine 30 lösbar verbunden sind. Der Behälter 17 ist mit seinem Behälterboden 33 an der Universalbohrmaschine 30 lösbar befestigt. Anstelle eines Behälterdeckels ist ein Lagergehäuse 34 im Behälter 17 angebracht, worin das Schneidwerkzeug 16 bezüglich seiner Rotationsbewegung um die Längsachse 2 beispielsweise mittels Kugellager 35 gelagert und mittels einer ringförmigen Dichtung (37) der Behälter (17) gegenüber der Umgebung abgedichtet ist. Zudem ist der Stutzen 18 für den Anschluss eines Vakuumschlauches an der Seitenwand des Behälters 17 angebracht. Zur Abdichtung des flexiblen Schaftes 8 ist in dessen Bohrung 10 ein Gummi- oder Kunststoffschlauch 36 entlang der Längsachse 2 eingeführt.

## Patentansprüche

1. Vorrichtung zur Gewinnung von Knochenspänen, welche
A) ein rotierbares Schneidwerkzeug (16) mit einer Längsachse (2), einem Schneidkopf (1) und einem an den Schneidkopf (1) anschliessenden, konzentrisch zur Längsachse (2) angeordneten longitudinalen Schaft (8); und
B) Antriebsmittel (14), welche dem Schneidwerkzeug (16) mit dem Schneidkopf (1) eine Rotationsbewegung um die Längsachse (2) aufprägen, umfasst; wobei
C) das Schneidwerkzeug (16) eine in Richtung der Längsachse (2) durchgehende Bohrung (10) aufweist und der Schneidkopf (1) mit mindestens einer Durchgangsöffnung (7) versehen ist, so dass die vom Schneidkopf (1) spanabhebend abgetragenen Knochenspäne durch die Bohrung (10) förderbar sind,
D) der Schaft (8) mindestens auf einem Teil seiner Länge bezüglich Torsion und Biegung um die Längsachse (2) elastisch ist; **dadurch gekennzeichnet, dass**
E) in der Bohrung (10) des Schneidwerkzeuges (16) entlang der Längsachse (2) zusätzlich ein Kunststoff- oder Gummischlauch (36) eingeführt ist.

2. Vorrichtung nach Anspruch 1, **dadurch gekennzeichnet, dass** der Schaft (8) aus einem spiralförmig gewickelten Metallband gefertigt ist.

3. Vorrichtung nach Anspruch 1, **dadurch gekennzeichnet, dass** der Schaft (8) aus einem Metallrohr mit balgartiger Wand besteht.

4. Vorrichtung nach einem der Ansprüche 1 bis 3, **dadurch gekennzeichnet, dass** der Schneidkopf (1) hohlzylindrisch gestaltet ist und einen sich entlang der Längsachse erstreckenden Hohlraum (9), einen mit einer Bohrspitze (20) und mit mindestens einer Schneidkante (3) versehenen vorderen Abschnitt (4), einen hohlzylindrischen hinteren Abschnitt (5) und mindestens die im vorderen Abschnitt (4) die Aussenwand (29) des Schneidkopfes (1) radial durchdringende Durchgangsöffnung (7) zur Förderung der durch die mindestens eine Schneidkante (3) abgetragenen Knochenspäne in den Hohlraum (9) des Schneidkopfes (1) umfasst.

5. Vorrichtung nach Anspruch 4, **dadurch gekennzeichnet, dass** die Bohrspitze (20) des Schneidkopfes (1) als Kugelkalottensektor ausgebildet ist.

6. Vorrichtung nach Anspruch 4, **dadurch gekennzeichnet, dass** die Bohrspitze (20) des Schneidkopfes (1) kugelkalottenförmig mit mindestens zwei koaxial und radial zur Längsachse (2) sich In den Hohlraum (9) erstreckenden Durchgangsöffnungen (7) ausgebildet ist, wobei an den Kanten der Durchgangsöffnungen (7) Schneidkanten (3) zum Abtragen von Knochenspänen angebracht sind und die abgetragenen Knochenspäne durch die Durchgangsöffnungen (7) in den Hohlraum (9) des Schneidkopfes (1) förderbar sind.

7. Vorrichtung nach einem der Ansprüche 1 bis 6, **dadurch gekennzeichnet, dass** die Antriebsmittel (14) aus einer Universalbohrmaschine (30) bestehen.

8. Vorrichtung nach einem der Ansprüche 1 bis 7, **dadurch gekennzeichnet, dass** das Schneidwerkzeug (16) durchgehend hohlzylindrisch ausgeführt ist, Mittel (13) zur Aufnahme eines Drehmomentes, welches eine Rotation des Schaftes (8) um die Längsachse (2) verursacht, aufweist und an seinem am Schneidkopf (1) anschliessbaren Ende (12) so an den hinteren Abschnitt (5) anschliessbar ist, dass die Bohrung (10) des hohlzylindrischen Schaftes (8) mit dem Hohlraum (9) fluchtend ausrichtbar ist und vom Schaft (8) das Drehmoment auf den Schneidkopf (1) übertragbar ist.

9. Vorrichtung nach Anspruch 8, **dadurch gekennzeichnet, dass** die Antriebsmittel (14) Einspannmittel (15) zur rotativen und axialen Fixierung der Mittel (13) am Schneidwerkzeug (16) umfassen.

10. Vorrichtung nach einem der Ansprüche 1 bis 9, **dadurch gekennzeichnet, dass** die Vorrichtung einen unter Vakuum stehenden Behälter (17) umfasst und das Schneidwerkzeug (16) an seinem vom Schneidkopf (1) entfernten Ende (21) auch während der spanabhebenden Gewinnung von Knochenspänen an den Behälter (17) anschliessbar ist.

11. Vorrichtung nach Anspruch 10, **dadurch gekennzeichnet, dass** der Behälter (17) fluchtend zur Längsachse (2) am Antriebsmittel (14) lösbar angebracht ist, wobei der Behälter (17) relativ zur Längsachse (2) stillsteht und das vom Schneidkopf (1) entfernte Ende (21) des rotierenden hohlzylindrischen Schneidwerkzeuges (16) mittels einer ringförmigen Dichtung (37) abgedichtet In den Behälter (17) mündet, so dass der Übergang zwischen Schneidwerkzeug (16) und Behälter (17) gegenüber der Umgebung luftdicht abgeschlossen ist.

12. Vorrichtung nach Anspruch 10 oder 11, **dadurch gekennzeichnet, dass** am Behälter (17) ein Stutzen (18) für den Anschluss eines Vakuumschlauches angebracht ist.

13. Vorrichtung nach einem der Ansprüche 10 bis 12, **dadurch gekennzeichnet, dass** der Behälter (17) eine Abscheidevorrichtung (19) zur Abscheidung der Knochenspäne aus dem Luftstrom umfasst.

14. Vorrichtung nach Anspruch 13, **dadurch gekennzeichnet, dass** die Abscheidevorrichtung (19) aus einem Filter besteht.

## Claims

1. An apparatus for harvesting bone grafts comprising
A) a rotatable cutting tool (16) with a longitudinal axis (2), a cutting head (1), and a longitudinal shaft (8) adjoining the cutting head (1) and extending concentrically to the longitudinal axis (2); and
B) drive means (14) which confer to the cutting tool (16) equipped with the cutting head (1) a rotatory movement about the longitudinal axis (2); with
C) the cutting tool (16) having a bore (10) penetrating it in the direction of the longitudinal axis (2), and the cutting head (1) being provided with at least one through hole (7) so that the bone chips removed by the cutting action of the cutting head (1) may be conveyed through the bore (10); and
D) the shaft (8) is resilient at least on part of its length with respect to twisting and bending,
**characterized in that**
E) the bore (10) of the cutting tool (16) is additionally lined with a plastic or rubber flexible tube (36) extending along the longitudinal axis (2).

2. An apparatus as claimed in claim 1, **characterised in that** the shaft (8) is made of a spirally wound metal strip.

3. An apparatus as claimed in claim 1, **characterised in that** the shaft (8) is made of a metal tube with a bellows-type side wall.

4. An apparatus as claimed in any of the claims 1 to 3, **characterised in that** the cutting head (1) is realised in the form of a hollow cylinder and comprises a cavity (9) extending along the longitudinal axis, a front segment (4) provided with a cutting tip (20) and with at least one cutting edge (3), a hollow, cylindrical rear segment (5), and at least the through hole (7) radially penetrating the outside wall (29) of the cutting head (1) in the front segment (4) and serving for conveying the bone chips removed by the at least one cutting edge (3) to the cavity (9) of the cutting head (1).

5. An apparatus as claimed in claim 4, **characterised in that** the cutting tip (20) of the cutting head (1) is realised in the form of a calotte sector.

6. An apparatus as claimed in claim 4, **characterised in that** the cutting tip (20) of the cutting head (1) is realised in the form of a calotte having at least two through holes (7) extending coaxially and radially to the longitudinal axis (2) into the cavity (9), the edges of the through holes (7) being provided with cutting edges (3) for removing bone chips and the removed bone chips being conveyable through the through holes (7) to the cavity (9) of the cutting head (1).

7. A device as claimed in any of the claims 1 to 6, **characterised in that** the drive means (14) consist of a universal drilling machine (30).

8. An apparatus as claimed in any of the claims 1 to 7, **characterised in that** the cutting tool (16) is realised, over its entire length, in the form of a hollow cylinder, that it comprises means (13) for receiving a turning moment which causes a rotation of the shaft (8) about the longitudinal axis (2), and that with its end portion (12) connectable to the cutting head (1) it may be connected to the rear segment (5) in such a way that the bore (10) of the hollow, cylindrical shaft (8) may be aligned flush with the cavity (9) and that the turning moment may be transmitted from the shaft (8) to the cutting head (1).

9. An apparatus as claimed in claim 8, **characterised in that** the drive means (14) comprise chucks (15) for locking the means (13) provided on the cutting tool (16) against rotative and axial displacement.

10. An apparatus as claimed in any of the claims 1 to 9, **characterised in that** the apparatus comprises a chamber (17) in which a vacuum is created and that the cutting tool (16) on its end portion (21) located opposite to the cutting head (1) is connectable to said chamber (17) even while the cutting and harvesting of bone chips is in progress.

11. An apparatus as claimed in claim 10, **characterised in that** the chamber (17) in alignment with the longitudinal axis (2) is releasably connected to the drive means (14), the chamber (17) being immobile with respect to the longitudinal axis (2) and the end portion (21) of the rotating, hollow, cylindrical cutting tool (16) located opposite to the cutting head (1) leading to said chamber (17), the point of connection between the two being sealed by an annular sealing so that the transition point between the cutting tool (16) and the chamber (17) is tightly sealed against the ambient air.

12. An apparatus as claimed in claim 10 or 11, **characterised in that** the chamber (17) is provided with a connection piece (18) for connecting a suction hose.

13. An apparatus as claimed in any of the claims 10 to 12, **characterised in that** the chamber (17) comprises a separator (19) for separating the bone chips from the air flow.

14. An apparatus as claimed in claim 13, **characterised in that** the separator (19) consists of a filter.

## Revendications

1. Dispositif de prélèvement de copeaux d'os, lequel comprend
A) un outil de coupe rotatif (16) ayant un axe longitudinal (2), une tête coupante (1) et une tige (8) longitudinale dans le prolongement de la tête coupante (1), disposée concentriquement à l'axe longitudinal (2) ; et
B) des moyens d'entraînement (14) qui imposent à l'outil de coupe (16) avec la tête coupante (1) d'effectuer un mouvement de rotation autour de l'axe longitudinal (2) ; où
C) l'outil de coupe (16) présente un trou traversant (10) dans le sens de l'axe longitudinal (2) et la tête coupante (1) est pourvue d'au moins une ouverture de passage (7), de sorte que les copeaux d'os prélevés par enlèvement de copeaux avec la tête coupante (1) peuvent être évacués à travers le trou (10) ;
D) la tige (8), au moins sur une partie de sa longueur, est élastique en termes de torsion et de flexion par rapport à l'axe longitudinal (2) ;
**caractérisé en ce que**
E) un tuyau de matière plastique ou de caoutchouc (36) est inséré en plus dans le trou (10) de l'outil de coupe (16) le long de l'axe longitudinal (2).

2. Dispositif selon la revendication 1, **caractérisé en ce que** la tige (8) est faite d'un ruban métallique enroulé en spirale.

3. Dispositif selon la revendication 1, **caractérisé en ce que** la tige (8) est faite d'un tube métallique à paroi annelée.

4. Dispositif selon l'une quelconque des revendications 1 à 3, **caractérisé en ce que** la tête coupante (1) est conçue sous forme de cylindre creux et comprend un espace creux (9) s'étendant le long de l'axe longitudinal, un segment avant (4) pourvu d'une pointe de perçage (20) et d'au moins une arête coupante (3), un segment arrière en forme de cylindre creux (5) et au moins une ouverture de passage (7) traversant radialement la paroi externe (29) de la tête coupante (1) dans le segment avant (4) pour évacuer les copeaux d'os prélevés avec la au moins une arête coupante (3) dans l'espace creux (9) de la tête coupante (1).

5. Dispositif selon la revendication 4, **caractérisé en ce que** la pointe de perçage (20) de la tête coupante (1) est conçue sous forme de segment d'une calotte sphérique.

6. Dispositif selon la revendication 4, **caractérisé en ce que** la pointe de perçage (20) de la tête coupante (1) est conçue sous forme de segment d'une calotte sphérique avec au moins deux ouvertures de passage (7) s'étendant coaxialement et radialement à l'axe longitudinal (2) dans l'espace creux (9), dans lequel sont disposées des arêtes coupantes (3) sur les bords des ouvertures de passage (7) pour prélever des copeaux d'os, et les copeaux d'os prélevés peuvent être évacués à travers les ouvertures de passage (7) dans l'espace creux (9) de la tête coupante (1).

7. Dispositif selon l'une quelconque des revendications 1 à 6, **caractérisé en ce que** les moyens d'entraînement (14) consistent en une perceuse universelle (30).

8. Dispositif selon l'une quelconque des revendications 1 à 7, **caractérisé en ce que** l'outil de coupe (16) est conçu sous forme de cylindre creux ininterrompu, présente des moyens (13) pour la réception d'un couple de rotation, lequel provoque une rotation de la tige (8) autour de l'axe longitudinal (2), et peut, au niveau de son extrémité (12) pouvant être reliée à la tête coupante (1), être reliée au segment arrière (5) de telle manière que le trou (10) de la tige en forme de cylindre creux (8) peut être mise en affleurement avec l'espace creux (9) et que le couple de rotation peut être transmis à la tête coupante (1) par la tige (8).

9. Dispositif selon la revendication 8, **caractérisé en ce que** les moyens d'entraînement (14) comprennent des moyen de serrage (15) pour bloquer les moyens (13) sur l'outil de coupe (16) en rotation et sur l'axe.

10. Dispositif selon l'une quelconque des revendications 1 à 9, **caractérisé en ce que** le dispositif comprend un récipient (17) sous vide et l'outil de coupe (16) peut, au niveau de son extrémité (21) éloignée de la tête coupante (1), être relié au récipient (17), même pendant le prélèvement de copeaux d'os par enlèvement de copeaux.

11. Dispositif selon la revendication 10, **caractérisé en ce que** le récipient (17) est relié de manière amovible en alignement avec l'axe longitudinal (2) aux moyens d'entraînement (14), le récipient (17) restant fixe par rapport à l'axe longitudinal (2) et l'extrémité (21) de l'outil de coupe rotatif en forme de cylindre creux (16) éloignée de la tête coupante (1) débouchant dans le récipient (17) au moyen d'un joint d'étanchéité annulaire (37), de sorte que la zone de transition entre l'outil de coupe (16) et le récipient (17) est ainsi hermétique vers l'extérieur.

12. Dispositif selon la revendication 10 ou 11, **caractérisé en ce qu'**un raccord (18) est disposé sur le récipient (17) pour le raccordement d'un tuyau à vide.

13. Dispositif selon l'une quelconque des revendications 10 à 12, **caractérisé en ce que** le récipient (17) comprend un dispositif de séparation (19) pour séparer les copeaux d'os du courant d'air.

14. Dispositif selon la revendication 13, **caractérisé en ce que** le dispositif de séparation (19) consiste en un filtre.
